(19) Europäisches Patentamt
European Patent Office
Office européen des brevets

(11) EP 4 273 119 A1

# (12) EUROPÄISCHE PATENTANMELDUNG

(43) Veröffentlichungstag:
**08.11.2023 Patentblatt 2023/45**

(21) Anmeldenummer: **22171287.0**

(22) Anmeldetag: **03.05.2022**

(51) Internationale Patentklassifikation (IPC):
***C07C 45/50*** *(2006.01)* ***C07C 47/02*** *(2006.01)*

(52) Gemeinsame Patentklassifikation (CPC):
(C-Sets verfügbar)
**C07C 45/50** (Forts.)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Evonik Operations GmbH**
**45128 Essen (DE)**

(72) Erfinder:
- **FRIDAG, Dirk**
  **45721 Haltern am See (DE)**
- **KNOSSALLA, Johannes**
  **46514 Gahlen (DE)**
- **BRÄCHER, Alexander**
  **45721 Haltern am See (DE)**
- **KUCMIERCZYK, Peter**
  **44628 Herne (DE)**
- **SALE, Anna Chiara**
  **45657 Recklinghausen (DE)**
- **FRANKE, Robert**
  **45772 Marl (DE)**
- **MARKOVIC, Ana**
  **45721 Haltern am See (DE)**

(74) Vertreter: **Evonik Patent Association**
**c/o Evonik Industries AG**
**IP Management**
**Bau 1042A/PB 15**
**Paul-Baumann-Straße 1**
**45772 Marl (DE)**

## (54) VERFAHREN ZUR HERSTELLUNG VON C5-ALDEHYDEN

(57) Gegenstand der Erfindung ist ein Verfahren zur Herstellung von C5-Aldehyden durch Hydroformylierung von Butenen mit Synthesegas in Anwesenheit eines homogen Katalysatorsystems und eines Lösemittels. Das Verfahren zeichnet sich dadurch aus, dass die Aldehydkonzentration in der Reaktionsmischung begrenzt wird.

EP 4 273 119 A1

(52) Gemeinsame Patentklassifikation (CPC): (Forts.)

C-Sets
**C07C 45/50, C07C 47/02**

## Beschreibung

Die vorliegende Erfindung betrifft ein Verfahren zur Herstellung von C5-Aldehyden durch Hydroformylierung von Butenen mit Synthesegas in Anwesenheit eines homogen Katalysatorsystems und eines Lösemittels. Das Verfahren zeichnet sich dadurch aus, dass die Aldehydkonzentration in der Reaktionsmischung begrenzt wird.

Die Hydroformylierung von Olefinen ist eine bekannte chemische Reaktion zur Herstellung von Aldehyden. In der chemischen Industrie werden mit Hydroformylierungsreaktionen weltweit jedes Jahr Millionen Tonnen Aldehyde hergestellt. Bei der Hydroformylierung werden die Olefine in Anwesenheit eines homogen Katalysatorsystem, das ein Übergangsmetall, beispielsweise Rhodium oder Cobalt, und einen Liganden umfassen kann, mit Synthesegas (Mischung aus CO und $H_2$) zu Aldehyden umgesetzt.

Ein Teil der Hydroformylierungsverfahren wird als sogenanntes Kreisgasverfahren durchgeführt, bei dem ein gasförmiger, produkthaltiger Austrag aus der Reaktionszone entnommen wird. Kreisgasverfahren werden üblicherweise für die Hydroformylierung kürzerer Olefine, d. h. Olefine mit maximal 5 Kohlenstoffatomen, eingesetzt, da die gebildeten Aldehyde ausreichend flüchtig sind, um mit der Gasphase die Reaktionszone verlassen zu können.

Das Problem bei Kreisgasverfahren ist, dass die Liganden, durch die bei der Hydroformylierung vorherrschenden Bedingungen abgebaut werden können. Die Abbaureaktionen, bei denen die Liganden zerstört werden, können verschiedene Prozesse sein, z. B. Hydrolyse, Oxidation, Abramov-Reaktion, Umesterung, die auch gleichzeitig nebeneinander ablaufen können. Während der Hydroformylierung kommt es dann zu einem kontinuierlichen Verlust an Liganden. In so einem Fall muss der Ligand dauerhaft und kontinuierlich nachdosiert werden, um weiter wirtschaftlich, d. h. mit ausreichenden Reaktionsumsätzen, arbeiten zu können.

Die Aufgabe der vorliegenden Erfindung bestand darin ein Verfahren zur Hydroformylierung bereitzustellen, bei dem die Abbaureaktionen des Liganden reduziert oder ganz vermieden werden. Eine weitere Aufgabe der vorliegenden Erfindung war die Bereitstellung eines solchen Verfahrens, bei dem kein großer präparativer oder anlagentechnischer Aufwand getrieben werden muss, um das Ziel, also die Reduzierung oder Vermeidung der Abbaureaktionen des Liganden, zu erreichen.

Überraschenderweise wurde gefunden, dass durch ein Absenken der Aldehydkonzentration in der Reaktionsphase weniger Ligand nachdosiert werden muss und das Verfahren trotzdem wirtschaftlich durchgeführt werden kann. Die Abbaureaktionen des Liganden werden offensichtlich verringert.

Die Aufgabe konnte mit einem Verfahren nach Anspruch 1 gelöst werden. Bevorzugte Ausführungsformen sind in den Unteransprüchen angegeben. Die vorliegende Erfindung betrifft demnach ein Verfahren zur Herstellung von C5-Aldehyden durch Hydroformylierung von Butenen, bei der die Butene mit Synthesegas in mindestens einer Reaktionszone in Gegenwart eines homogen Katalysatorsystems und eines Lösemittels umgesetzt werden und bei dem aus der Reaktionszone ein Kreisgas entnommen wird, welches zumindest einen Teil der Produktaldehyde, nicht umgesetzte Butene und Butane enthält, dadurch gekennzeichnet, dass die Aldehydkonzentration in der flüssigen Reaktionsmischung im Reaktor durch das Kreisgas unter 15 Masse % gehalten wird.

Die Absenkung der Aldehydkonzentration wird im Rahmen der vorliegenden Erfindung durch Anheben der Kreisgasmengen erreicht. Technisch sind aber auch andere Maßnahmen denkbar. Durch die Erhöhung der Kreisgasmenge lässt sich erreichen, dass die gebildeten Produktaldehyde schneller aus der mindestens einen Reaktionszone entfernt werden. Zudem werden die oben genannten Abbaureaktionen des Liganden reduziert. Das erfindungsgemäße Verfahren lässt sich also so betreiben, dass im Vergleich zu konventionellen Verfahren bei ähnlichen Umsätzen weniger Ligand nachdosiert werden muss. Das Verfahren ist also ressourcenschonender als vergleichbare Verfahren. Außerdem werden Kosten gespart, weshalb das erfindungsgemäße Verfahren insgesamt wirtschaftlicher betrieben werden kann.

Bei der erfindungsgemäßen Hydroformylierung werden als Edukte Butene, d. h. lineare Butene (n-Buten, also 1-Buten und 2-Buten) und/oder verzweigte Butene (iso-Buten), eingesetzt, die dann mit Synthesegas (Mischung aus CO und $H_2$) in Gegenwart eines homogenen Katalysators zu den gewünschten C5-Aldehyden umgesetzt werden. Die zu hydroformylierenden Butene können in reiner Form eingesetzt oder in technisch verfügbaren Mischungen als Einsatzgemisch für die erfindungsgemäße Hydroformylierung bereitgestellt werden. Der Begriff Einsatzgemisch ist so zu verstehend, dass er jegliche Art von butenhaltigen Gemischen betrifft, die die Butene in einer Menge enthalten, die es ermöglicht, die Hydroformylierung wirtschaftlich zu betreiben. Solche Einsatzgemisches enthalten neben Butenen auch die entsprechenden Alkane, also Butane.

Technische Gemische, die Butene und Butane enthalten und als Einsatzgemisch für das vorliegende Verfahren eingesetzt werden können, sind Leichtbenzinfraktionen aus Raffinerien, C4-Fraktionen aus FC- oder Steamcrackern, Gemische aus Fischer-Tropsch-Synthesen, Gemische aus der Dehydrierung von Butanen und durch Metathese oder aus anderen technischen Prozessen entstandene Gemische. Für das erfindungsgemäße Verfahren geeignete Butengemische können aus der C4-Fraktion eines Steamcrackers gewonnen werden, beispielsweise das Raffinat I , das Raffinat II oder das Raffinat III. Ein anderes butenhaltiges Gemisch, welches in der vorliegenden Erfindung als Einsatzgemisch eingesetzt werden kann, ist das Rohbutan. Es ist auch möglich 1-Buten als Einsatzgemisch einzusetzen. Erfindungsgemäß bevorzugte butenhaltige Gemische zur Verwendung als Einsatzgemisch sind Kohlenwasserstoffströme, die einen Butengehalt von mindestens 15 Gew.-% aufweisen. In einer bevorzugten Ausführungsform der vorliegenden Erfindung sind die butenhaltigen Gemisch zur Verwendung als Einsatzgemisch frei von Dienen und Alkinen, d.h. der Gehalt von Dienen und Alkinen liegt jeweils unter 100 ppm, vorzugsweise unter 10 ppm, besonders bevorzugt unter 1 ppm.

Durch die erfindungsgemäße Hydroformylierung von Butenen lassen sich Aldehyde mit fünf Kohlenstoffatomen (C5-Aldehyde) herstellen. Zu den C5-Aldehyden gehören n-Pentanal (Valeraldehyd), iso-Pentanal (Isovaleradelhyd), sec-Pentanal (2-Methylbutanal) und tert-Pentanal (Pivalaldehyd). In einer bevorzugten Ausführungsform der vorliegenden Erfindung ist das beschriebene Verfahren ein Verfahren zur Herstellung von n-Pentanal (Valeraldehyd).

Das erfindungsgemäße Verfahren wird außerdem unter Verwendung eines Lösemittels durchgeführt, in dem das homogene Katalysatorsystem gelöst vorliegt. Zumindest das Lösemittel und das darin gelöste homogene Katalysatorsystem bilden das Reaktionsgemisch, welches als flüssige Phase in dem mindestens einen Reaktor vorliegt In einer bevorzugten Ausführungsform weist das Lösemittel einen höheren Siedepunkt als die eingesetzten Butene und die Produktaldehyde auf. Als Lösemittel können dem Fachmann bekannte Lösemittel eingesetzt werden. Als Lösemittel für das erfindungsgemäße Verfahren können insbesondere INB (Isononylbenzoat) oder DINCH (1,2-Cyclohexandicarbonsäurediisononylester) eingesetzt werden.

Das bei dem erfindungsgemäßen Verfahren eingesetzte Katalysatorsystem umfasst oder besteht aus einem Metall aus der 8. oder 9. Gruppe des Periodensystems der Elemente und mindestens einen organischen Phosphorenthaltenden Liganden. Das Metall ist ausgewählt aus Eisen, Ruthenium, Iridium, Cobalt und Rhodium. Bevorzugte Metalle für das erfindungsgemäße Katalysatorsystem sind Cobalt und Rhodium ist, besonders bevorzugt ist Rhodium. Als Ligand werden vorzugsweise Mono- oder Biphosphitliganden eingesetzt, die dem Fachmann grundsätzlich bekannt sind. Geeignete Liganden sind beispielsweise in den Patentanmeldungen WO 2017/080690 A1, WO 2014/056732 A1, WO 2014/056735 A1, WO 2014/056736 A1, WO 2014/056737 A1 und DE 10 2008 002 187 A1 offenbart. Ein besonders bevorzugter Ligand ist eine Verbindung der Formel (1):

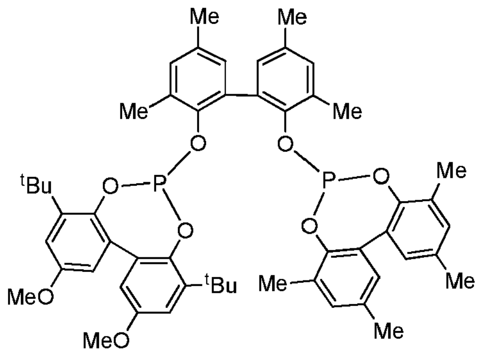

(1).

In einer besonders bevorzugten Ausführungsform wird bei dem erfindungsgemäßen Verfahren ein Katalysatorsystem umfassend Rhodium und einen Ligand nach der oben gezeigten Formel (1) eingesetzt.

Das molare Verhältnis des Rhodiums zu den Mono- oder Bisphosphit-Liganden (Ligand/Rhodium-Verhältnis) liegt vorzugsweise in einem Bereich von 1 bis 100. Auf jedes Rhodium kommen demnach 1 bis 100 Mono- oder Bisphosphit-Liganden. Das Ligand/Rhodium-Verhältnis liegt weiterhin bevorzugt im Bereich von 1 bis 20, besonders bevorzugt liegt es im Bereich von 1 bis 2. Die Konzentration des Rhodiums im flüssigen Reaktionsgemisch liegt in einem Bereich von 1 bis 1000 Massen-ppm, insbesondere in einem Bereich von 20 bis 300 Massen-ppm und ganz besonders im Bereich von 40 bis 150 Massen-ppm.

Das homogene Katalysatorsystem wird vorzugsweise nicht als einsatzbereiter, aktiver Komplex in das Verfahren eingebracht, sondern wird in situ, also im Reaktor herstellt werden. Hierfür wird der aktive Komplex innerhalb des mindestens einen Reaktors in Gegenwart Liganden aus Rhodiumverbindungen als Precursor hergestellt. Geeignete Rhodiumverbindungen dafür sind zum Beispiel Rhodium(II)- und Rhodium(III)-salze, wie Rhodium(III)chlorid, Rhodium(III)nitrat, Rhodium(III)sulfat, Kalium-Rhodiumsulfat, Rhodium(II)- bzw. Rhodium(III)-carboxylat, Rhodium(II)- und Rhodium(III)acetat, Rhodium(II)octanoat, Rhodium(II)nonanoat, Rhodium(III)oxid, Salze der Rhodium(III)säure, Trisammoniumhexachlororhodat(III). Weiterhin eignen sich Rhodiumkomplexe, wie Rhodiumbiscarbonylacetylacetonat, Acetylacetonatobisethylenrhodium(I). Besonders geeignet sind Rhodiumacetat, Rhodiumoctanoat und Rhodiumnonanoat.

In einer besonders bevorzugten Ausführungsform der vorliegenden Erfindung wird die Hydroformylierung zusätzlich in Anwesenheit eines Stabilisators durchgeführt. Der Stabilisator ist vorzugsweise eine organische Aminverbindung, besonders bevorzugt eine organische Aminverbindung, die mindestens eine 2,2,6,6-Tetramethylpiperidineinheit nach Formel (I) enthält:

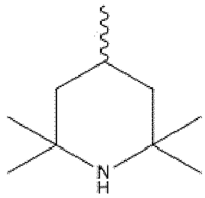

(I)

In einer besonders bevorzugten Ausführungsform der vorliegenden Erfindung ist der Stabilisator ausgewählt aus der Gruppe, bestehend aus den Verbindungen der nachfolgenden Formeln (I.1), (I.2), (I.3), (I.4), (1.5), (I.6), (I.7) und (I.8).

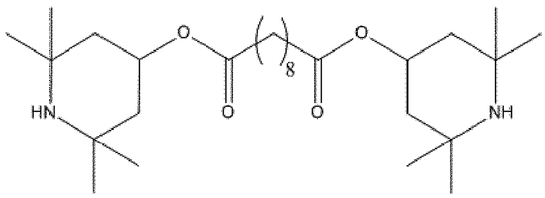

(I.1);

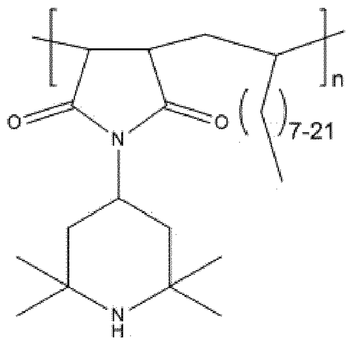

(I.2),

wobei n einer ganzen Zahl von 1 bis 20 entspricht;

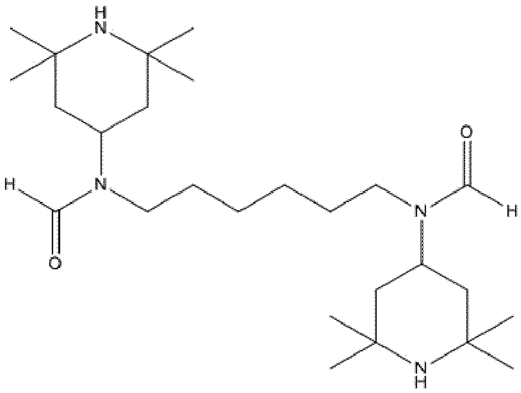

(I.3);

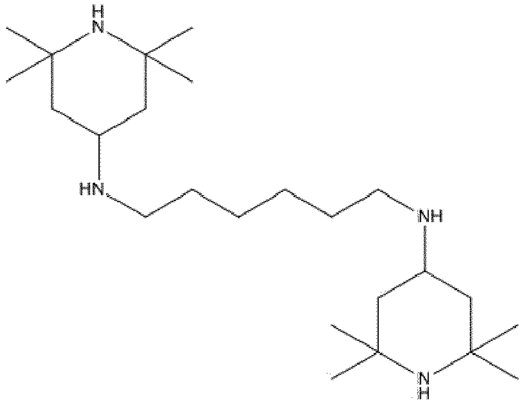

(I.4);

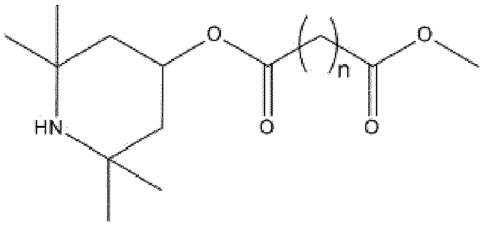

(I.5),

wobei n einer ganzen Zahl von 1 bis 12 entspricht;

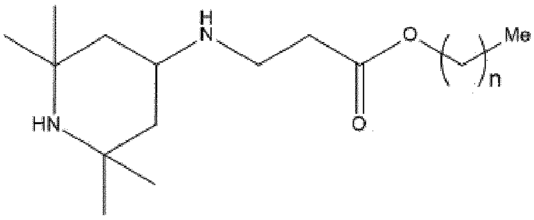

(I.6),

wobei n einer ganzen Zahl von 1 bis 17 entspricht;

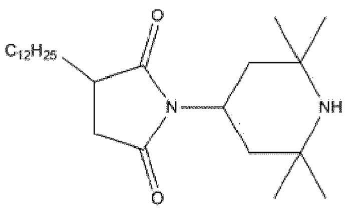

(I.7);

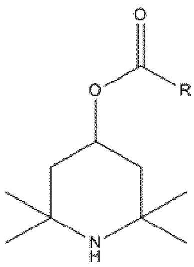

(I.8),

wobei R einer C6- bis C20-Alkylgruppe entspricht.

Ganz besonders bevorzugt handelt es sich bei dem organischen Amin um ein Sebacinsäuredi-4-(2,2,6,6-tetramethylpiperidinyl)-ester, der beispielsweise ist unter dem Markennamen Tinuvin® 770 DF erhältlich ist. Das molare

Verhältnis der Liganden zu dem Stabilisator liegt vorzugsweise im Bereich von 0.1 zu 10 bis 10 zu 1, insbesondere in einem Bereich von 5 zu 10 bis 10 zu 5 und ganz besonders in einem Bereich von 0.8 zu 1 bis 1 zu 0.8.

Das Verfahren gemäß der vorliegenden Erfindung wird in mindestens einer Reaktionszone, vorzugsweise in einer einzigen Reaktionszone durchgeführt. Eine Reaktionszone umfasst im Sinne der vorliegenden Erfindung mindestens einen Reaktor, vorzugsweise mehr als einen Reaktor. Die einzelnen Reaktoren innerhalb der Reaktionszone können parallel oder in Reihe geschaltet vorliegen. In einer besonders bevorzugten Ausführungsform weist die Reaktionszone mindestens zwei parallel geschaltete Reaktoren auf.

In dem mindestens einen Reaktor liegt eine flüssige Phase vor, die zumindest das Lösemittel und das darin gelöste homogene Katalysatorsystem umfasst. Ist ein Stabilisator anwesend liegt dieser ebenfalls in der flüssigen Phase in dem mindestens einen Reaktor vor. Der mindestens eine Reaktor umfasst auch eine gasförmige Phase. Die Reaktion findet aber in der flüssigen Phase statt, da dort auch das Katalysatorsystem vorhanden ist. Die Zugabe der Edukte, bzw. des Einsatzgemisches, welches die Butene und die Butane enthält, und des Synthesegases erfolgt vorzugsweise am Boden des Reaktors, insbesondere über geeignete und dem Fachmann bekannte Vorrichtungen wie Düsen. Die Zugabe erfolgt in eines besonders bevorzugten Ausführungsform der vorliegenden Erfindung mengengeregelt

Das hier beschriebene Hydroformylierungsverfahren kann grundsätzlich kontinuierlich oder diskontinuierlich durchgeführt werden. Vorzugsweise wird das Verfahren aber kontinuierlich durchgeführt.

Die typischen Bedingungen bei der Hydroformylierung sind dem Fachmann. Die Hydroformylierung wird vorzugsweise bei einer Temperatur im Bereich von 65 bis 200 °C, weiterhin bevorzugt bei einer Temperatur im Bereich von 75 bis 175 °C und besonders bevorzugt bei einer Temperatur im Bereich von 85 bis 135 °C, durchgeführt. Der Druck beträgt bei der Hydroformylierung vorzugsweise 10 bis 200 bar, weiterhin bevorzugt 12,5 bis 100 bar, besonders bevorzugt 15 bis 25 bar.

Wie erwähnt wird bei dem erfindungsgemäßen Verfahren ein Kreisgas, welches zumindest einen Teil der Produktaldehyde, nicht umgesetzte Butene und Butane enthält, aus der Reaktionszone bzw. aus dem mindestens einen Reaktor entnommen. Das entnommene Kreisgas kann anschließend zu einem Aerosolabscheider geführt werden, um mitgerissene und im Kreisgas enthaltene flüssige Schwebeteilchen aus dem Kreisgas zu entfernen. Das im Aerosolabscheider aus dem Kreisgas abgetrennte flüssige Gemisch kann zur Reaktionszone bzw. dem oder den Reaktor(en) zurückgeführt werden. Der Aufbau und die Betriebsweise eines Aerosolabscheiders sind dem Fachmann geläufig. Zur besseren Abscheidung der Aerosole können diese Abscheider auch mit einer Waschflüssigkeit beaufschlagt werden oder entsprechende Waschkolonnen, wie sie ebenfalls dem Fachmann geläufig sind, eingesetzt werden.

Das Kreisgas kann nach dem Aerosolabscheider zu einem Kondensator geführt werden, wodurch die im Kreisgas enthaltenen Produktaldehyde, das nicht umgesetzte Buten, das Butan und das Lösemittel zumindest partiell auskondensiert werden. Der Kondensator wird vorzugsweise bei einer Temperatur unterhalb der Reaktionstemperatur betrieben. Die Temperatur im Kondensator kann zwischen 30 bis 100 °C betragen. Besonders bevorzugt ist eine Temperatur von 70 bis 90 °C. Das Synthesegas bleibt dabei in der Gasphase und wird nicht auskondensiert. Im Kondensator sind dann also eine flüssige Phase, in der die zumindest partiell auskondensierten Bestandteile, also die Produktaldehyde, das nicht umgesetzte Buten, das Butan und das Lösemittel, enthalten sind, und eine Gasphase, die das Synthesegas umfasst, vorhanden.

Die auskondensierte flüssige Phase und die Gasphase können in einem Phasentrenner voneinander getrennt werden. Die erhaltene Gasphase wird vorzugsweise über einen Kreisgasverdichter zur Reaktionszone bzw. dem oder den Reaktoren zurückgeführt. Die auskondensierte flüssige Phase wird vorzugsweise einer Rohproduktabtrennung unterworfen, die aus einer oder mehreren Destillationskolonnen bestehen kann. Dabei werden die nicht umgesetzten Butene und die Butane (Rest-C4) vom Rohprodukt, welches zumindest die gebildeten Aldehyde und ggf. hochsiedende Nebenprodukte und/oder das Lösemittel enthält, abgetrennt. Dem Fachmann sind die Bedingungen für die Destillation bekannt. Das Rohprodukt kann dann in nachfolgenden Reaktionsschritten eingesetzt werden.

Sofern INB als Lösemittel eingesetzt wird, kann für die vollständige Abtrennung des Lösemittels eine zusätzliche Lösemittelabtrennung vorteilhaft und gegebenenfalls notwendig sein. INB ist vergleichsweise leicht flüchtig, was bei einer Erhöhung der Kreisgasmenge dazu führt, dass verstärkt INB mit dem Kreisgas ausgetragen wird. Das INB kann in nachfolgenden Verfahrens- oder Reaktionsschritten jedoch zu Problemen führen und muss daher abgetrennt werden. Die dazu einsetzbare Lösemittelabtrennung besteht aus mindestens einer Destillationskolonne. Dabei wird das Rohprodukt aus der Rohproduktabtrennung eingesetzt und das Lösemittel und ggf. die hochsiedenden Nebenprodukte von den C5-Aldehyden abgetrennt. Das Lösemittel kann dann zurück zum Reaktor geführt werden. Sofern hochsiedende Nebenprodukte vorhanden sind, kann es vorteilhaft sein einen Purge, also ein Ausschleusen eines Teils des Rezyklats vorzusehen, um die hochsiedenden Nebenprodukte nicht im Reaktor anzureichern.

Es ist aber auch möglich die Rohprodukteabtrennung und die Lösemittelabtrennung in einer einzigen Destillationskolonne vorzusehen. Das ist beispielsweise mit einer Trennwandkolonne möglich, wo das Rest-C4 am Kopf anfällt, das Lösemittel und ggf. die hochsiedenden Nebenprodukte im Sumpf anfallen und die C5-Aldehyde dazwischen abgenommen werden können.

Wird DINCH als Lösemittel eingesetzt, ist eine zusätzliche Lösemittelabtrennung nicht erforderlich. DINCH hat

einen höheren Siedepunkt und ist weniger flüchtig als INB. Trotz einer Erhöhung der Kreisgasmenge wird kein oder zumindest nicht mehr Lösemittel mit dem Kreisgas ausgetragen.

Der erhaltene Produktaldehyd kann in einer bevorzugten Ausführungsform einem nachfolgenden Reaktionsschritt, beispielsweise einer Hydrierung, einer Aldolisierung (Aldolkondensation), einer Oxidation zu einer Carbonsäure oder einer Umsetzung zum Amin unterworfen werden.

Ein weiterer Gegenstand im Rahmen der vorliegenden Erfindung ist ein flüssiges Gemisch, umfassend ein Aldehyd, Cobalt oder Rhodium, einen organischen Phosphor-enthaltenden Liganden und ein Lösemittel, wobei die Aldehydkonzentration in dem flüssigen Gemisch unter 15 Masse-% liegt. Als Lösemittel können dem Fachmann bekannte Lösemittel eingesetzt werden. Als Lösemittel für das erfindungsgemäße Verfahren können insbesondere INB (Isononylbenzoat) oder DINCH (1,2-Cyclohexandicarbonsäurediisononylester) eingesetzt werden. Als Ligand werden vorzugsweise Phosphoresterverbindungen, insbesondere Mono- oder Biphosphitliganden eingesetzt, die dem Fachmann grundsätzlich bekannt sind. Geeignete Liganden sind oben bereits genannt worden und meinen insbesondere einen Liganden nach der Formel (1). Dieses Gemisch ist insbesondere das flüssigen Reaktionsgemisch aus dem erfindungsgemäßen Verfahren zur Herstellung von C5-Aldehyden.

In der Fig. 1 wird das erfindungsgemäße Verfahren exemplarisch erläutert. Das Verfahren findet in einem Reaktionsbehälter (1) statt, in dem eine flüssige Reaktionsphase (I), bestehend aus Lösemittel und dem homogen gelösten Katalysatorsystem, vorliegt. Der Reaktionsbehälter (1) enthält auch eine freie Gasphase (II) und kann zudem über einen internen Wärmetauscher (16) geheizt oder gekühlt werden. Es werden kontinuierlich Synthesegas (VI) und Edukt (V), also das eingesetzte Olefin bzw. ein olefinhaltiger Kohlenwasserstoffstrom, in den In den Reaktionsbehälter (1) gefahren, wobei das Edukt (V) wahlweise in gasförmiger oder flüssiger Form zugegeben werden kann. Dem Synthesegas (VI) kann wahlweise zusätzlich Wasserstoff (VII) zugegeben werden. Weiterhin kann dem Reaktionsbehälter (1) eine Ligandenlösung (IV) und/oder das Lösemittel (III) in flüssiger Form zugeführt werden, um eventuelle Verluste auszugleichen.

Über das Kreisgassystem, welches in diesem Schema aus einem Verdichter (13), einem Aerosolabscheider (7), einem Kondensator (8), einem Phasentrennbehälter (9) und einem Rohrleitungssystem (6,11), welches die Komponenten des Kreisgassystems miteinander verbindet, wird ein kontinuierlicher Gasstrom aus dem Reaktionsbehälter (1) entnommen. Dieser Gasstrom wird durch den Verdichter (13) aufgebaut und kann aktiv verändert werden. Der Gasstrom wird von unten in die flüssige Reaktionsphase (I) geleitet. Das zugeführte Edukt (V) und das Synthesegas (VI) reagieren in der flüssigen Phase zu den entsprechenden Aldehyden. Über deren Partialdruck gehen die entstandenen Produkte und nicht umgesetzte Edukte in die Gasphase (II) über und verlassen den Reaktionsbehälter (1). Der entnommene Gasstrom wird anschließend durch einen Aerosolabscheider (7) geleitet, in dem die mitgerissenen Anteile der Reaktionsphase (I) abgeschieden und wieder in den Reaktionsbehälter (1) zurückgeleitet werden. Der erhaltene Gasstrom gelangt dann zu einem nachfolgenden Kondensator (8), wo die Temperatur so weit abgesenkt wird, dass die verbliebenden Edukte und Produkte zumindest teilweise aus der Gasphase auskondensiert. Die dadurch entstehende flüssige Phase (produkthaltig) und die gasförmige Phase werden zusammen in den Phasentrennbehälter (9) geleitet. Dort werden die produkthaltige Flüssigphase und Gasphase voneinander getrennt. Über die Rohrleitung (10) wird das flüssige Rohprodukt (IX) zur weiteren Aufarbeitung geleitet. Die Gasphase aus dem Phasentrennbehälter (9) wird auf die Saugseite des Verdichters (13) geleitet und zurück zum Reaktionsbehälter (1) geführt. Der Anlagendruck kann mit der Rohrleitung (14) über das Abgas (IIX) geregelt werden.

Die vorliegende Erfindung wird nachfolgend anhand von Beispielen erläutert. Diese Beispiele sind nur eine Veranschaulichung der vorliegenden Erfindung und nicht einschränkend zu verstehen.

Beispiel 1 (nicht erfindungsgemäß)

Vor Start der Reaktion wurde das gesamte System mit Stickstoff inertisiert und anschließend mit Synthesegas stickstofffrei (kleiner 5% $N_2$) gespült und anschließend bis auf 14bar (abs.) mit Synthesegas aufgedrückt. Insgesamt wurden in den Reaktionsbehälter vor Start etwa 30$m^3$ Reaktionsphase vorgelegt.

Diese wurde zuvor wie folgt angesetzt. Jeweils 5 $m^3$ Isononylbenzoat (INB) wurden in einem inertisierten Ansatzbehälter für 5h mit Stickstoff durchspült, um gelösten Sauerstoff zu entfernen. Als nächstes wurden 140kg Tinuvin 770DF, 130 kg Ligand mit der in Formel (1) gezeigten Struktur und 11 kg Acetylacetonato(dicarbonyl)rhodium(I) über eine mit Stickstoff inertisierte Pulverschleuse zum INB gegeben. Dieser Ansatz wurde auf 60°C aufgeheizt und nachdem sich der Feststoff aufgelöst hatte, wurde der Inhalt in den Reaktionsbehälter gefahren. Die Verfolgung der Ligandenkonzentration in der Reaktionsphase ist durch eine Probennahme aus dem Reaktionssystem mit anschließender Analytik über eine HPLC Methode möglich. Bei dieser Methode kann ebenfalls der oxidierte Anteil des Liganden bestimmt werden. Der Oxidationsanteil lag unter 10% der eingesetzten Ligandenmenge.

Im nächsten Schritt wurde der Kreisgasverdichter angefahren. Die Reaktionsphase wurde auf 120°C aufgeheizt. Der spätere Reaktionsdruck vom 17bar (abs) wurde über ein Regelventil im Abgasstrom eingestellt. Überschüssiger Druck im Reaktionssystem wurde während der Aufheizphase über diesen Weg abgebaut.

Nach Erreichen der Reaktionstemperatur wurde die Eduktdosierung mit 4500 kg/h gestartet. Das Synthesegas wurde in einem stöchiometrischem Verhältnis von 1:1.2 zum Butengehalt im Edukt gefahren. Als Edukt kam ein C4-Strom mit einer Zusammensetzung von 35% Butenen (Mischung aus 1 und 2 Butenen) und 65% n-Butan zum Einsatz.

Durch die Zugabe des Eduktes und durch die Reaktion erhöhte sich das Volumen der Reaktionsphase auf 50 $m^3$. Um den Stand auf einem konstanten Niveau zu halten, wurde das Kreisgas entsprechend angepasst. Der Anteil des nicht im Komplex gebundenen Liganden wurde durch regelmäßige Probenahme und Analytik mittels HPLC bestimmt und durch Oxidation abgebauter Ligand wurde durch Dosierung von Liganden ersetzt. Dazu wurde unter inerten Bedingungen der Ligand und Tinuvin 770 DF im C4-freien Rohaldehyd gelöst und in die Reaktionsphase gefahren. Dadurch wurde das Verhältnis zwischen Rhodium in der Reaktion und dem nichtgebundenen Liganden konstant gehalten.

Aus der nachgefahrenen Masse Ligand und der produzierten Masse an Aldehyd ergibt sich ein Ligandeneinsatzfaktor in kg Ligand pro Tonne hergestelltem Aldehyd. Der Einsatzfaktor Ligand berechnet sich wie folgt:

$$Einsatzfaktor\ Ligand\ \frac{kg}{t} = \frac{dosierte\ Masse\ Ligand\ als\ Reinsubstanz\ in\ kg}{produzierte\ Masse\ Aldehyd\ in\ t}$$

Dieser wird in diesem Beispiel gleich 100% unter diesen Bedingungen gesetzt und dient als Referenz für Beispiel 2. Gleichzeitig wurde aus der Probe, mittels gaschromatographischer Analyse, der Aldehydgehalt in der Reaktionslösung bestimmt. Im Mittel betrug der Aldehydgehalt 15 Masse%.

Aus den stündliche hergestellten Molen Aldehyd und stündlich eingesetzten Molen Butene wurde zudem die Ausbeute nach der folgenden Formel errechnet. Es stelle sich eine Ausbeute von 60% ein.

$$y\ Aldehyde = \frac{mol/h\ Aldehyde\ im\ kondensierten\ Ausgang\ der\ Anlage}{mol/h\ Butene\ im\ Feed\ der\ Anlage - mol/h\ Butene\ im\ auskondiserten\ Ausgang\ der\ Anlage}$$

Beispiel 2 (erfindungsgemäß)

Der Versuch wurde analog zu Beispiel 1 angesetzt und gestartet. Allerdings wurde die Kreisgasmenge so gefahren, dass die Aldehydkonzentration in der Reaktionsphase im Mittel 13% betrug. Standverluste wurden durch Nachfahren von Lösemittel, in diesem Fall INB, ausgeglichen.

Analog zum Beispiel 1 wurden auch in diesem Beispiel die Anteile des freien Liganden und die Ausbeute bestimmt. Wie auch schon im Beispiel 1 wird der Ligandeneinsatzfaktor in kg pro Tonne hergestelltem Aldehyd bestimmt. Der Ligandeneinsatzfaktor betrug nur 75% bezogen auf Beispiel 1. Eine Übersicht über die ermittelten Daten findet sich in Tabelle 1.

Tabelle 1: Übersicht über die Reaktionsdaten

| | Beispiel 1 (nicht erfindungsgemäß) | Beispiel 2 (erfindungsgemäß) |
|---|---|---|
| Einsatzfaktor | 100 % | 75 % |
| Aldehydkonzentration in der Reaktionsphase | 15% | 13 % |
| Ausbeute | 60% | 55 % |

## Patentansprüche

1. Verfahren zur Herstellung von C5-Aldehyden durch Hydroformylierung von Butenen, bei der die Butene mit Synthesegas in mindestens einer Reaktionszone in Gegenwart eines homogen Katalysatorsystems und eines Lösemittels umgesetzt werden, wobei aus der Reaktionszone ein Kreisgas entnommen wird, welches zumindest einen Teil der Produktaldehyde, nicht umgesetzte Butene und Butane enthält, **dadurch gekennzeichnet, dass** die Aldehydkonzentration in der flüssigen Reaktionsmischung im Reaktor durch das Kreisgas unter 15 Masse-% gehalten wird.

2. Verfahren nach Anspruch 1, wobei das aus der Reaktionszone entnommene Kreisgas zunächst zu einem Aerosolabscheider geführt wird.

3. Verfahren nach Anspruch 2, wobei das Kreisgas nach dem Aerosolabscheider zu einem Kondensator geführt wird,

wodurch die im Kreisgas enthaltenen Produktaldehyde, nicht umgesetzte Butene und das Lösemittel auskondensiert werden.

4. Verfahren nach Anspruch 3, wobei die kondensierte, flüssige Phase und die Gasphase in einem Phasentrenner voneinander getrennt werden.

5. Verfahren nach Anspruch 4, wobei die Gasphase über einen Kreisgasverdichter zur Reaktionszone zurückgeführt wird.

6. Verfahren nach Anspruch 4 oder 5, wobei die kondensierte flüssige Phase einer C4/C5-Trennung geführt wird, die aus einer oder mehreren Destillationskolonnen besteht.

7. Verfahren nach Anspruch 6, wobei die vorher abgetrennten Produktaldehyde und das Lösemittel in einer Lösemittelabtrennung voneinander getrennt werden und das Lösemittel zurück zum Reaktor geführt wird.

8. Verfahren nach einem der Ansprüche 1 bis 7, wobei das Lösemittel einen höheren Siedepunkt als die Produktaldehyde aufweist.

9. Verfahren nach einem der Ansprüche 1 bis 8, wobei das Lösemittel INB (Isononylbenzoat) oder DINCH (1,2-Cyclohexandicarbonsäurediisononylester) ist.

10. Verfahren nach einem der Ansprüche 1 bis 9, wobei die Hydroformylierung in der Reaktionszone zusätzlich in Anwesenheit eines Stabilisators stattfindet.

11. Verfahren nach Anspruch 10, wobei der Stabilisator eine organische Aminverbindung ist, die mindestens eine 2,2,6,6-Tetramethylpiperidineinheit nach Formel (I) enthält:

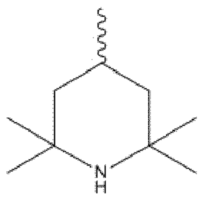

.

12. Verfahren nach einem der Ansprüche 1 bis 11, wobei das homogene Katalysatorsystem ein Metall aus der 8. oder 9. Gruppe des Periodensystems der Elemente und mindestens einen organischen Phosphor-enthaltenden Liganden umfasst, vorzugsweise einen Mono- oder Biphosphitliganden.

13. Verfahren nach Anspruch 12, wobei das Metall Eisen, Ruthenium, Iridium, Cobalt oder Rhodium, vorzugsweise Cobalt und Rhodium ist.

14. Verfahren nach einem der Ansprüche 1 bis 13, wobei das homogene Katalysatorsystem Rhodium und einen Liganden mit der Struktur nach Formel (1) umfasst:

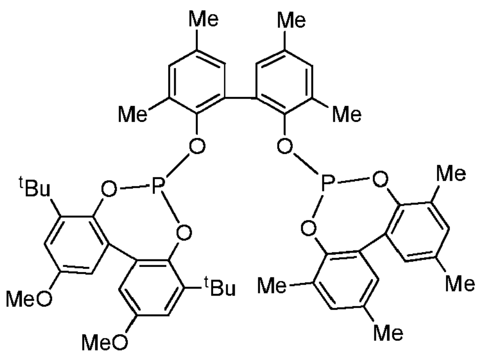

(1).

**15.** Flüssiges Gemisch, umfassend ein Aldehyd, Cobalt oder Rhodium, einen organischen Phosphor-enthaltenden Liganden und ein Lösemittel, wobei die Aldehydkonzentration in dem flüssigen Gemisch unter 15 Masse-% liegt.

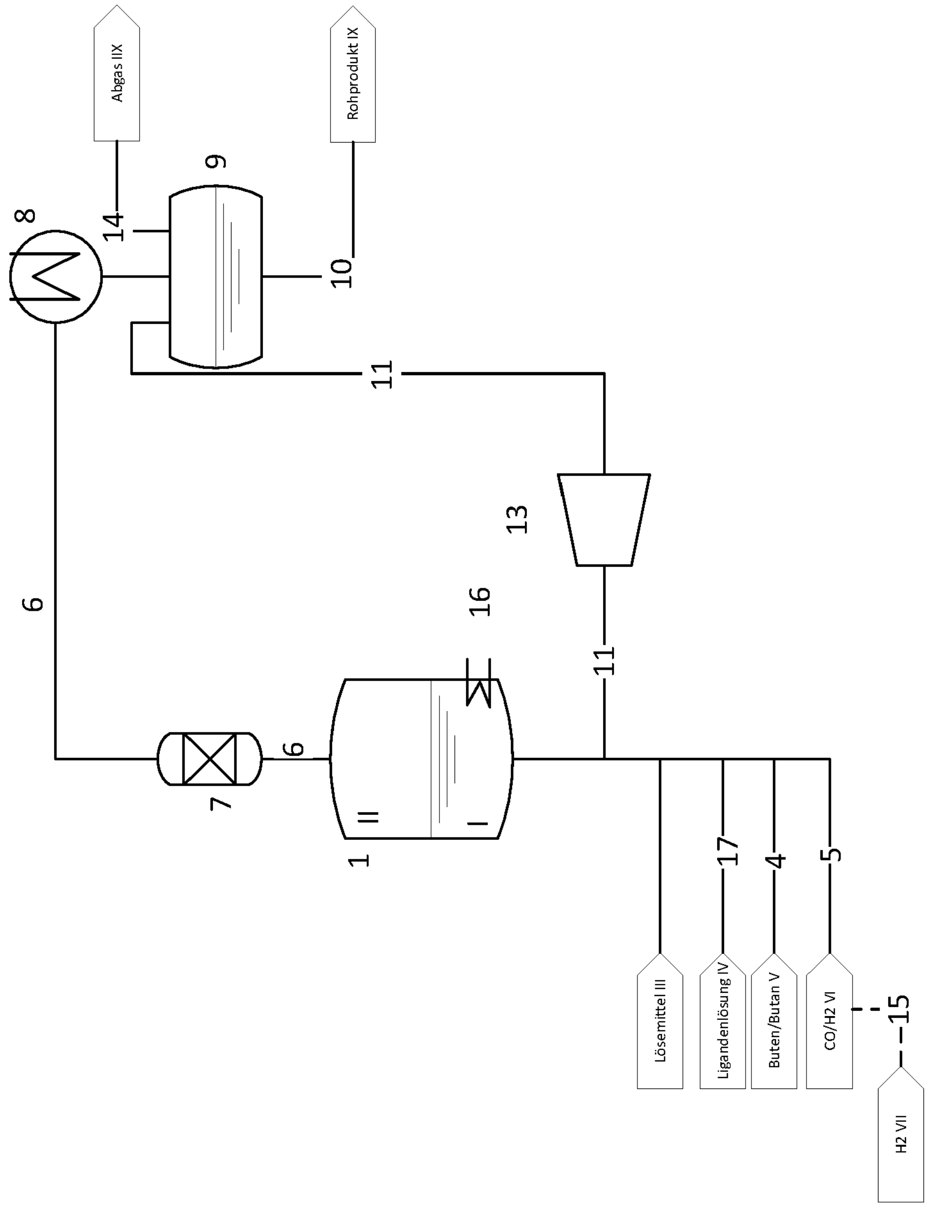
Abgas IIX
Rohprodukt IX
8
9
14
10
11
6
13
16
11
6
7
1
II
I
17
4
5
Lösemittel III
Ligandenlösung IV
Buten/Butan V
CO/H2 VI
15
H2 VII

Fig. 1

Europäisches Patentamt
European Patent Office
Office européen des brevets

# EUROPÄISCHER RECHERCHENBERICHT

Nummer der Anmeldung
EP 22 17 1287

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X,D | WO 2014/056732 A1 (EVONIK INDUSTRIES AG [DE]; FRIDAG DIRK [DE] ET AL.) 17. April 2014 (2014-04-17) * Seite 20, Zeilen 4-8; Beispiele 1-4 * * Seite 15, Zeile 17 - Seite 16, Zeile 20 * ----- | 1-15 | INV. C07C45/50 C07C47/02 |
| | | | RECHERCHIERTE SACHGEBIETE (IPC) |
| | | | C07C |

Der vorliegende Recherchenbericht wurde für alle Patentansprüche erstellt

1

| Recherchenort | Abschlußdatum der Recherche | Prüfer |
|---|---|---|
| Den Haag | 28. Oktober 2022 | Matés Valdivielso, J |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.........................................................................
& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 22 17 1287

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

28-10-2022

| Im Recherchenbericht angeführtes Patentdokument | Datum der Veröffentlichung | Mitglied(er) der Patentfamilie | Datum der Veröffentlichung |
|---|---|---|---|
| WO 2014056732 A1 | 17-04-2014 | AR 092987 A1 | 13-05-2015 |
| | | AR 092988 A1 | 13-05-2015 |
| | | AR 092989 A1 | 13-05-2015 |
| | | AR 092990 A1 | 13-05-2015 |
| | | CA 2887107 A1 | 17-04-2014 |
| | | CA 2887565 A1 | 17-04-2014 |
| | | CA 2887580 A1 | 17-04-2014 |
| | | CA 2887582 A1 | 17-04-2014 |
| | | CN 104781225 A | 15-07-2015 |
| | | CN 104837851 A | 12-08-2015 |
| | | CN 104837852 A | 12-08-2015 |
| | | CN 104837853 A | 12-08-2015 |
| | | CN 104854118 A | 19-08-2015 |
| | | EP 2802550 A1 | 19-11-2014 |
| | | EP 2906571 A1 | 19-08-2015 |
| | | EP 2906572 A1 | 19-08-2015 |
| | | EP 2906573 A1 | 19-08-2015 |
| | | EP 2906574 A1 | 19-08-2015 |
| | | ES 2566069 T3 | 08-04-2016 |
| | | ES 2603929 T3 | 02-03-2017 |
| | | ES 2614055 T3 | 29-05-2017 |
| | | ES 2615677 T3 | 07-06-2017 |
| | | JP 6246218 B2 | 13-12-2017 |
| | | JP 6335905 B2 | 30-05-2018 |
| | | JP 2015531402 A | 02-11-2015 |
| | | JP 2015536302 A | 21-12-2015 |
| | | JP 2015536303 A | 21-12-2015 |
| | | JP 2015536912 A | 24-12-2015 |
| | | JP 2016500676 A | 14-01-2016 |
| | | KR 20150065869 A | 15-06-2015 |
| | | KR 20150065886 A | 15-06-2015 |
| | | KR 20150067322 A | 17-06-2015 |
| | | KR 20150070224 A | 24-06-2015 |
| | | KR 20150070225 A | 24-06-2015 |
| | | SG 11201502756P A | 28-05-2015 |
| | | SG 11201502777V A | 28-05-2015 |
| | | SG 11201502815P A | 28-05-2015 |
| | | SG 11201502824V A | 28-05-2015 |
| | | SG 11201502843S A | 28-05-2015 |
| | | TW 201422632 A | 16-06-2014 |
| | | US 2015224488 A1 | 13-08-2015 |
| | | US 2015266008 A1 | 24-09-2015 |
| | | US 2015273455 A1 | 01-10-2015 |
| | | US 2015274627 A1 | 01-10-2015 |
| | | US 2015290633 A1 | 15-10-2015 |
| | | WO 2014056732 A1 | 17-04-2014 |

EPO FORM P0461

Für nähere Einzelheiten zu diesem Anhang : siehe Amtsblatt des Europäischen Patentamts, Nr.12/82

## ANHANG ZUM EUROPÄISCHEN RECHERCHENBERICHT ÜBER DIE EUROPÄISCHE PATENTANMELDUNG NR.

EP 22 17 1287

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

28-10-2022

| Im Recherchenbericht angeführtes Patentdokument | Datum der Veröffentlichung | Mitglied(er) der Patentfamilie | Datum der Veröffentlichung |
|---|---|---|---|
| | | WO 2014056733 A1 | 17-04-2014 |
| | | WO 2014056735 A1 | 17-04-2014 |
| | | WO 2014056736 A1 | 17-04-2014 |
| | | WO 2014056737 A1 | 17-04-2014 |
| | | ZA 201503226 B | 25-05-2016 |
| | | ZA 201503227 B | 27-01-2016 |
| | | ZA 201503228 B | 21-12-2016 |
| | | ZA 201503232 B | 27-01-2016 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- WO 2017080690 A1
- WO 2014056732 A1
- WO 2014056735 A1
- WO 2014056736 A1
- WO 2014056737 A1
- DE 102008002187 A1